# EUROPEAN PATENT APPLICATION

(11) **EP 3 838 142 A1**
(43) Date of publication of application: **23.06.2021**
(21) Application number: 19219733.3
(22) Date of filing: 26.12.2019
(51) Int. Cl.: A61B 5/11, A61B 5/16, A61B 5/00

(54) **METHOD AND DEVICE FOR MONITORING DEMENTIA-RELATED DISORDERS**

(30) Priority: 20.12.2019 PT 2019116013
(71) Applicant: Associação Fraunhofer Portugal Research, 4200-135 Porto (PT)
(72) Inventor: GUIMARÃES, VÂNIA, 4200-135 PORTO (PT)
(74) Representative: Patentree

(57) **Abstract**

The present disclosure relates to a method and device for monitoring dementia-related disorders, in particular monitoring gait and/or cognitive function decline. The device and method disclosed assesses dementia-related disorders of a person by monitoring cognitive task decline and walking gait changes as the person performs dual tasks in comparison with single-tasks.

## Description

### TECHNICAL FIELD

The present disclosure relates to a method and device for monitoring dementia-related disorders, in particular monitoring gait and/or cognitive function decline.

### BACKGROUND

Gait requires the coordination of multiple systems, including all levels of the nervous system, many parts of the musculoskeletal system, and the cardiorespiratory system. Clinical studies suggest that the preferred walking speed can be used as a sensitive marker of general health, fall risk, disability and cognitive decline. Preferred walking speed is also able to indicate early signs of dementia (1).

Gait is typically assessed in clinical settings using a wide variety of tests. However, parameters obtained through these tests are likely to be unrepresentative of the real walking performance due to the brevity of the tests and interferences such as the presence of the clinician. With gait patterns being also influenced by personality, mood and sociocultural factors (1), there are still no clearly accepted standards (methods and cut-offs) for the assessment of gait in the elderly (3).

Even though the majority of the studies recognize the importance of gait speed assessment as a marker of health, declines in gait speed may not be the primary effect that is noticed when an underlying disease starts to develop. In fact, anatomically similar brain regions mediate gait and cognitive functions. Therefore, when capacity to perform both functions simultaneously decrease, capacity may be allocated to one task over the other, negatively influencing gait, cognition or both simultaneously. Different attention allocation strategies may be used in different dual-task situations. Thus, capturing both physical and cognitive functional status can help reflect a more implicit functional status of the individual. The majority of the studies report the dual-task interference just as a dual-task effect on gait, neglecting the effects on the cognitive task. However, when considered in isolation, wrong conclusions may be drawn (5).

Dual-task interference may be a more sensitive marker for cognitive and/or gait decline. Most of the activities of the daily living involve the simultaneous performance of two or more tasks, making it possible to capture dual-task interference during free-living conditions. Currently, there are no solution to assess dual-task interference without resorting to specific clinical tests. Assessments in free-living conditions may be more representative of the health status of the individuals (3), thus enabling early detection of underlying disorders. Early assessments may help inform treatment decisions and/or automatically set interventions for gait-cognitive training in older adults.

Document US 9693724 B2 discloses a system and method to unobtrusively quantify the effect of mobility, physical activity, learning, social interaction and diet on cognitive function. It assesses brain health of a person based on the person's use of one or more electronic devices. The brain health metric is a percentile rank relative to a population of users. There is no explicit reference to dual-task interference assessment, just individual metrics of an individual's interaction with an electronic device, e.g. applications opened, inputs typed, gesture patterns, body motions, eye movements, or voice input. Furthermore, the brain health metric reflects a rank relative to a population of users rather than an individual marker of changes in health.

Document US 8764532 B1 discloses a fall and concussion prediction system including a measurement assembly with a surface that is able to measure one or more quantities of the subject's contact with the surface. The system also includes a visual display device with a virtual reality scenario and a data processing device that receives the data based on the subject's contact with the surface and processes them to control the movement of at least one element of the interactive game in the visual display device. One or more performance parameters (e.g. balance, visual acuity, reaction time) are used to assess the probability of fall and concussion. The proposed solution requires the use of a system with a surface and display that is not designed to assess a subject in free-living conditions. Moreover, there is no explicit reference to gait, cognitive and dual-task assessment.

Document WO 2018050763 A1 discloses a method for assessing cognition and movement diseases or disorders from a dataset of cognition and/or fine motoric activity measurement obtained from the same subject using a mobile device. The invention also relates to a method for identifying whether a subject will benefit from a therapy for a cognition and movement disease or disorder. The present invention contemplates a mobile device comprising a processor, at least one sensor and a database as well as software which is tangibly embedded to said device. Fine motor skills are assessed using specific tasks like drawing shapes on the smartphone. There is no explicit reference to dual-task assessment.

Document US 20180070823 A1 discloses a system and method to assess cognitive function, including passive and active behavioral data. The method comprises receiving at the server sensor data from a wearable device and sending, by the server, instructions to the wearable device to present a cognitive test based on sensor data. The results are compared against a predetermined baseline. Physical parameters can be brain-wave activity, heart rate, body temperature, talking speed, reaction times, amount of physical motion, etc. The solution relies on explicit cognitive tests that are also used to evaluate the effectiveness of a treatment after a change in treatment dose. While cognitive assessment is based on tests, there is no explicit reference to dual-task assessments or gait assessment.

Document JP 2017518856 A discloses a system and method for enhancing the cognitive ability by customizing cognitive training plan for individuals. Customize cognitive training plan through difficulty progression. Application limited to cognitive training, without considering gait function assessment and training.

Document JP 2018512202 A discloses a cognitive function evaluation tool to evaluate individual's cognitive ability while performing multi-tasking. Multi-tasking refers to a visual motor task and perceptual reactive response, i.e. it requires a fine movement as a reaction to the stimulus. Assessments are not performed in free-living conditions. Moreover, multi-task assessment is limited to the assessment of the cognitive task; there is no reference to the dual task effect on gait and cognition.

Document US 7294107 B2 discloses a battery of tests for cognitive assessment, intended to be used by clinicians. No references to assessments in free-living conditions were made.

Document KR 101534831 B1 discloses an apparatus for recognizing and estimating dementia. The evaluation apparatus uses computer-based assessment tools and smartphone-based apps assessment. It has no explicit reference to assessments in free-living conditions, in particular with regards to the assessment of gait and dual-task interference.

Document KR 101205908 B1 discloses a cognitive impairment and dementia evaluation system based on ECG monitoring and walking assessment. No references to dual-task assessment and free-living conditions assessment were made.

Document US9958460B2 discloses biomarkers, diagnostic and prognostic methods for Alzheimer's disease and other neurodegenerative disorders. These biomarkers are solely based on biological measures taken from biological samples of the subject.

Document KR101709855B1 discloses a method for providing predictive information about Alzheimer's disease, relying on the analysis of MRI data. There is no reference to other input data.

Document EP3105345B1 discloses a method for predicting increased risk of Alzheimer's disease using genetic and biological data. There is no reference to other input data.

Document Montero-Odasso, M. et al. 2017 "Association of Dual-Task Gait With Incident Dementia in Mild Cognitive Impairment: Results From the Gait and Brain Study" found that dual-task gait testing could identify patients with mild cognitive impairment at risk of progression to dementia. Patients were followed up for 6 years, with biannual visits. The presented method does not evaluate the dual-task effect on the cognitive task, and does not refer evaluation in free-living conditions. Gait assessments were performed resorting to a gait test on the lab.

Document Buracchio, T. et al. 2010 "The Trajectory of Gait Speed Preceding Mild Cognitive Impairment" compared the trajectory of gait decline between those who developed mild cognitive impairment (MCI) and those who remained cognitively intact. They found that gait decline as indexed by gait speed accelerated up to 12 years prior to MCI. Evaluations were performed annually, and gait speed assessed using a fixed walking distance and a stopwatch. The article does not consider dual-task assessment neither evaluations in free-living conditions.

Document Dumurgier, J. et al. 2017 "Gait Speed and Decline in Gait Speed as Predictors of Incident Dementia" found that gait is slower up to 7 years prior to clinical onset of dementia. Gait speed over 6m was assessed 4 times over the follow-up using two photoelectric cells. The paper does not consider dual-task assessment neither evaluations in free-living conditions.

Document Rosso, A. et al. 2017 "Dual-task walking as a predictor for later cognitive impairment" assessed dual-task gait speed changes, and found that higher dual-task costs in gait speed was associated with greater risk of cognitive impairment 8 years later. Authors did not address dual-task costs on cognitive functions, and did not include measurements in free-living conditions.

Document KR20160005924A discloses a system to predict and recognize the signs and symptoms of dementia using a multichannel physiological signal sensing unit (incl. temperature, video, acceleration, respiration, among others). The patent does not refer dual-task assessments and does not include the use of a smartphone.

Document KR101205908B1 discloses an MCI and Alzheimer's disease evaluation system using gait dynamics and ECG monitoring, evaluated in daily life. The method is not explicitly targeting cognitive domains neither dual-task assessment.

Document Blumenthal, J. et al. 2017 "Cognitive capacity and smartphone dual-task gait measurement" developed a mobile application to perform quantitative gait and cognition assessment in the single and dual-task conditions. They implemented a game-like secondary task that users could perform simultaneously while walking with the mobile device fixed to their trunks. They implemented an auditory version of the Go/No-Go task, in which the user responds to "Go" by tapping on the screen of the smartphone or resists to tapping if the "No-Go" stimuli occurs. The presented approach is based on tests, rather than an opportunistic, pervasive, evaluation in free-living conditions.

These facts are disclosed in order to illustrate the technical problem addressed by the present disclosure.

### References

1. W. Pirker and R. Katzenschlager, "Gait disorders in adults and the elderly," Wien Klin Wochenschr, vol. 129, no. 3, pp. 81-95, 2017.
2. B. Salzman, "Gait and balance disorders in older adults," Am Fam Physician, vol. 82, no. 1, pp. 61-68, Jul. 2010.
3. M. Schimpl, C. Lederer, and M. Daumer, "Development and Validation of a New Method to Measure Walking Speed in Free-Living Environments Using the Actibelt® Platform," PLOS ONE, vol. 6, no. 8, p. e23080, Aug. 2011.
4. J. A. Cohen, J. Verghese, and J. L. Zwerling, "Cognition and gait in older people," Maturitas, vol. 93, pp. 73-77, Nov. 2016.
5. P. Plummer and G. Eskes, "Measuring treatment effects on dual-task performance: a framework for research and clinical practice," Front Hum Neurosci, vol. 9, Apr. 2015.

### GENERAL DESCRIPTION

The present disclosure relates to a method and device for monitoring dementia-related disorders, in particular monitoring gait and/or cognitive function decline.

The method and device of the present disclosure enables the unobtrusive monitoring of gait and/or cognitive capacity in free-living conditions, being therefore more representative of the real performance and functional status of the person. While existing methods are mostly based on tests to be performed at clinics, the proposed solution is not affected by the issues associated to the application of tests, such as interferences due to the test itself or interferences due to the presence of the clinician. Further, it enables the unobtrusive monitoring of gait and/or cognitive performance over time, enabling an early detection of decline and the establishment of preventive strategies. Current solutions compare the performance of the person with age and gender-matched characteristics, which may not represent the actual health status of the person as some characteristics may be influenced by personality, mood and sociocultural factors. The detection of changes in performance surpasses these influences, as the performance of the person is always compared with the individual's baseline performance.

The early detection of decline is particularly important to tackle dementia in our growing older population. Dementia can be caused by a variety of diseases and injuries that primarily or secondarily affect the brain, such as Alzheimer's disease or stroke. Dementia is one of the major causes of disability and dependency among older people worldwide. Alzheimer's disease is the most common form of dementia and may contribute to 60-70% of cases. Current treatments, however, can only modestly slow the progression of symptoms, and their efficacy depends on early administration. Due to the difficulty in detecting and diagnosing cognitive decline, a critical aspect of dementia care is early diagnosis. Diagnosis is often delayed, thus hindering treatment and impacting healthcare costs. Furthermore, drug development decisions also rely on sparse and subjective data which impacts clinical trial results and costs. The use of unobtrusive monitoring tools for cognitive functions presents an opportunity to advance healthcare through enhanced diagnosis, prognosis and personalized therapy management. The present disclosure provides a more cost-effective, efficient and less burdensome approach to diagnosis and monitor in clinical practice and clinical trials.

In an embodiment, mobile phone interaction data such as speech, texting and touch performance are collected and analysed to provide an index score of cognitive performance.

In an embodiment, speech data is collected from regular phone calls. The speech data collected is analysed and metrics of speech performance is generated from the data using speech signal processing techniques.

In an embodiment, the metrics of performance generated from the speech data are (1) acoustic metrics such as speaking rate, articulation rate, duration of silent speech pauses and (2) linguistic metrics such as synthetic complexity, lexical content and fluency errors.

In an embodiment, texting data is collected from keyboard interaction while texting on the phone in order to determine texting performance.

In an embodiment, the matrices of speech and texting performance is associated with a timestamp. Screen on or screen off timing is also monitored to assess time points at which the mobile phone is actually in use.

In an embodiment, the present disclosure describes an apparatus for the unobtrusive, pervasive assessment of the dual-task interference on gait and cognition in free-living conditions, the apparatus comprising:
an inertial sensor configured to detect and analyze the gait patterns of the person so as to obtain a gait score;
a cognition tracing module configured to evaluate the cognition of the person so as to obtain a cognition score;
a processing module configured to collect single-task and dual-task gait and cognition scores, and calculate the dual-task effects in gait and cognition.

In an embodiment, the processing module is configured to analyze the evolution of single and dual-task effects and detect changes in gait and/or cognition over time by comparing the performance with previous points in time.

In an embodiment, the processing module is configured to detect gait and cognitive decline and predict negative outcomes related to aging, such as dementia and falls.

In an embodiment, the inertial sensor is configured to analyze inertial sensor data or pressure data coming from smart shoes or inertial sensor data coming from wearables or the smartphone.

In an embodiment, the inertial sensor may further improve the accuracy of the gait analysis using location data from the mobile phone.

In an embodiment, the electronic data processor is configured to track cognition in multiple domains, resorting to the analysis of mobile phone usage data.

In an embodiment, changes in single-task and dual-task effects are used to automatically adjust personalized gait and cognition training plans and therapy.

In an embodiment, the device for assessing dementia-related disorders of a person by monitoring cognitive task decline and walking gait changes as the person performs dual tasks in comparison with single-tasks, is a personal mobile electronic device.

In an embodiment, the method of the present disclosure comprises:
calculating gait and cognition scores;
storing gait and cognition scores;
processing single-task and dual-task gait and cognition scores to calculate dual-task effects, and evaluate changes in dual-task effects over time;
setting personalized gait & cognition training plans and therapy.

In an embodiment, a computer program is designed for performing the method described in the present disclosure.

In an embodiment, the present disclosure describes a device for assessing dementia-related disorders of a person by monitoring cognitive task decline and walking gait changes as the person performs dual tasks in comparison with single-tasks, wherein the device is a personal mobile electronic device, and the cognitive task is typing or speaking on the device,
a single-task being defined as either walking or performing the cognitive task;
a dual-task being defined as simultaneously walking and performing the cognitive task;
the device comprising:
an inertial sensor for collecting gait patterns of the person;
a touch input sensor for collecting touch-typing data or a microphone for collecting speech data;
an electronic data processor configured for:
   acquiring gait patterns from the inertial sensor and acquiring touch-typing data from the touch input sensor and/or speech data from the microphone,
   analysing the acquired gait patterns so as to obtain a single-task gait score and a dual-task gait score;
   analysing the acquired touch-typing data and/or speech data so as to obtain a single task cognition score and a dual-task cognition score;
   calculating a gait dual-task effect, gait DTE, score as a proportion between the dual-task gait score and the single-task gait score;
   calculating a cognition dual-task effect, cognition DTE, score as a proportion between the dual-task cognition score and the single-task cognition score;
   using the gait DTE and cognition DTE for assessing dementia-related disorders.

In an embodiment, the electronic data processor is further configured for tracking the gait DTE and cognition DTE over time for monitoring dementia-related disorders by determining changes in scores over a period of time.

In an embodiment, the device is for assessing cognitive decline and dementia; Alzheimer's disease and other related dementia; inclination to falls, instability, walking disability; gait and cognitive decline; or combinations thereof.

In an embodiment, the present disclosure describes a method for assessing dementia-related disorders of a person by monitoring cognitive task decline and walking gait changes as the person performs dual tasks in comparison with single-tasks, wherein the cognitive task is typing or speaking on a personal mobile electronic device,
a single-task being defined as either walking or performing the cognitive task;
a dual-task being defined as simultaneously walking and performing the cognitive task;
the device comprising:
an inertial sensor for collecting gait patterns of the person;
a touch input sensor for collecting touch-typing data or a microphone for collecting speech data;
an electronic data processor;
wherein the method comprises carrying out by the personal mobile electronic device the steps of:
   acquiring gait patterns from the inertial sensor and acquiring touch-typing data from the touch input sensor and/or speech data from the microphone,
   analysing the acquired gait patterns so as to obtain a single-task gait score and a dual-task gait score;
   analysing the acquired touch-typing data and/or speech data so as to obtain a single task cognition score and a dual-task cognition score;
   calculating a gait dual-task effect, gait DTE, score as a proportion between the dual-task gait score and the single-task gait score;
   calculating a cognition dual-task effect, cognition DTE, score as a proportion between the dual-task cognition score and the single-task cognition score;
   using the gait DTE and cognition DTE for assessing dementia-related disorders.

In an embodiment, the method further comprises tracking the gait DTE and cognition DTE over time for monitoring dementia-related disorders by determining changes in scores over a period of time.

In an embodiment, the method is for assessing cognitive decline and dementia; Alzheimer's disease and other related dementia; inclination to falls, instability, walking disability; gait and cognitive decline; or combinations thereof.

In an embodiment, the present disclosure describes a non-transitory computer-readable storage medium including program instructions for implementing a device for assessing dementia-related disorders of a person by monitoring cognitive task decline and walking gait changes as the person performs dual tasks in comparison with single-tasks, the program instructions including instructions executable by a data processor to carry out the method disclosed.

### BRIEF DESCRIPTION OF THE DRAWINGS

The following figures provide preferred embodiments for illustrating the disclosure and should not be seen as limiting the scope of invention.
**Figure 1** illustrates dual-task effect calculation in light of the conceptual model proposed by Plummer, P. and Eskes, G. (2015) [5].
**Figure 2** shows a schematic overview of the method utilised.
**Figure 3** shows an example of the metrices obtained.
**Figure 4** shows an example of a decline in DTE over time.

### DETAILED DESCRIPTION

The present disclosure relates to a method, device and uses thereof for monitoring and determining gait and/or cognitive function decline.

In an embodiment, the disclosed method is for use the following: early detection of cognitive decline and dementia; prediction of Alzheimer's disease and other related dementia; prediction of falls, instability, disability and survival; decentralized continuous patient monitoring; personalised therapy management; prevention of gait and cognitive decline; decentralized patient monitoring in clinical trials.

In an embodiment, mobile phone interaction data such as speech, texting and touch performance are collected and analysed to provide an index of cognitive performance.

In an embodiment, speech data is collected from regular phone calls. The speech data collected is analysed and metrics of speech performance is generated from the data using speech signal processing techniques.

In an embodiment, the metrics of performance generated from the speech data are (1) acoustic metrics such as speaking rate, articulation rate, duration of silent speech pauses and (2) linguistic metrics such as synthetic complexity, lexical content and fluency errors.

In an embodiment, texting data is collected from keyboard interaction while texting on the phone in order to determine texting performance.

In an embodiment, the matrices of speech and texting performance is associated with a timestamp. Screen on or screen off timing is also monitored to assess time points at which the mobile phone is actually in use.

In an embodiment, single-task performance on gait and cognition (i.e. doing a single task in isolation) are analyzed against dual-task performance (i.e. doing both tasks simultaneously). Assessments are performed in an opportunistic and unobtrusive way, using the data recorded during free-living conditions. For example, 1) walking while not using the smartphone - gait performance assessment in single-task; 2) texting a message without walking - cognitive assessment in single-task; 3) Walking while texting a message - gait and cognitive assessment in dual-task. Dual-task interference or effect (DTE) on gait and cognition are calculated for different cognitive tasks, in view of the conceptual model proposed by Plummer, P. and Eskes, G. (2015) [**Error! Bookmark not defined**.], enabling an early detection of decline.

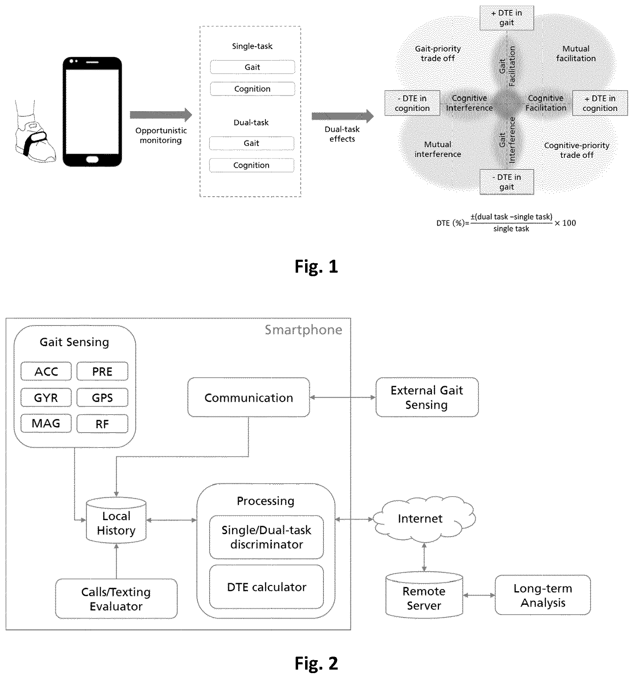

Figure 1 illustrates dual-task effect calculation in light of the conceptual model proposed by Plummer, P. and Eskes, G. (2015) [5].

Figure 2 shows a schematic overview of the method utilised.

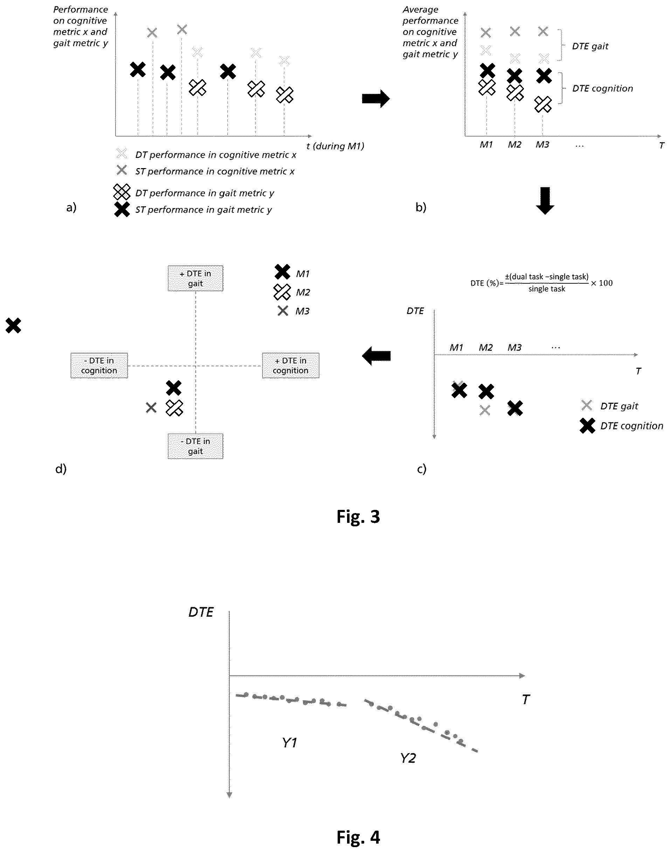

In an embodiment, the present disclosure describes a method to discriminate single from dual-task activities and automatically derive the corresponding performances on gait and cognition, where single task is "walking while not using the smartphone" (i.e. gait metrics are temporally aligned with not using the mobile phone) or "making a call or texting without walking" (i.e. gait and call/texting metrics are not temporally aligned) and dual-task is "walking and making a call or texting" (i.e. gait and call/texting metrics are temporally aligned). Timestamps of metrics extracted from walking, making a call and texting, and timestamps of using/not using the mobile phone, are used to discriminate single from dual-task activities (Figure 1 and Figure 3a).

In an embodiment, metrics extracted under single and dual-task conditions within a month are averaged out. The dual-task effect (DTE) is calculated for each extracted metric (Figure 3b and 3c).

In an embodiment, DTEs are mapped in a three-dimensional space, where DTE in gait and DTE in cognition for a pair of metrics are considered simultaneously and change with time (Figure 3d). Each pair of metrics is analyzed in the long-term, to detect long-term changes in gait and/or cognition. Linear regression analysis is used to assess in case the rate of decline begins to accelerate and determine the time when this change occurs, indicative of a more pronounced decline(Figure 5).In an embodiment, early detection of decline is attained by comparing DTE values with previous DTE values measured in a previous point in time. Only data from the individual is required to detect declines in gait and/or cognition.

In an embodiment, only normal usage interaction data are used to track cognitive performance during daily living. Specific smartphone applications may also be included for a more objective measurement of cognition (including specific cognitive domains, such as working memory, attention and executive functions) through specific tests that can be performed in single-task or in dual-task, i.e. executing the cognitive task alone or while walking. These applications may be presented as games to give the user a clearer purpose for the proposed tasks and at the same time increase motivation to perform these tasks, i.e. walking while playing (with walking being also part of the game).

In an embodiment, individual clinical markers of cognitive and/or gait decline can be used to predict gait and/or cognitive disorders, including dementia, as well as other negative outcomes related to ageing - falls, disability and survival.

In an embodiment, individual digital markers are used to design tailored preventive strategies to tackle cognitive and gait decline and adjust therapy according to the individual needs of the patient. Simultaneous stimulation of gait and cognitive aspects can be attained using exergames, which are presented on the smartphone, and played using a step-based interaction relying on an intelligent real-time analysis of the data collected from the Smart Shoes. Single and dual-task assessments performed in free-living conditions are used to understand the impact of the interventions on both aspects, and, at the same time, adjust interventions and therapy to be more efficient, taking into account the cognitive and gait domains that are mostly affected by dual-task interferences. (Figure 1)

In an embodiment, the present disclosure describes a device for assessing dementia-related disorders of a person by monitoring cognitive task decline and walking gait changes as the person performs dual tasks in comparison with single-tasks, wherein the device is a personal mobile electronic device, and the cognitive task is typing or speaking on the device,
a single-task being defined as either walking or performing the cognitive task;
a dual-task being defined as simultaneously walking and performing the cognitive task;
the device comprising:
an inertial sensor for collecting gait patterns of the person;
a touch input sensor for collecting touch-typing data or a microphone for collecting speech data;
an electronic data processor configured for:
   acquiring gait patterns from the inertial sensor and acquiring touch-typing data from the touch input sensor and/or speech data from the microphone,
   analysing the acquired gait patterns so as to obtain a single-task gait score and a dual-task gait score;
   analysing the acquired touch-typing data and/or speech data so as to obtain a single task cognition score and a dual-task cognition score;
   calculating a gait dual-task effect, gait DTE, score as a proportion between the dual-task gait score and the single-task gait score;
   calculating a cognition dual-task effect, cognition DTE, score as a proportion between the dual-task cognition score and the single-task cognition score;
   using the gait DTE and cognition DTE for assessing dementia-related disorders.

In an embodiment, the electronic data processor is further configured for tracking the gait DTE and cognition DTE over time for monitoring dementia-related disorders by determining changes in scores over a period of time.

In an embodiment, the device is for assessing cognitive decline and dementia; Alzheimer's disease and other related dementia; inclination to falls, instability, walking disability; gait and cognitive decline; or combinations thereof.

In an embodiment, the present disclosure describes a method for assessing dementia-related disorders of a person by monitoring cognitive task decline and walking gait changes as the person performs dual tasks in comparison with single-tasks, wherein the cognitive task is typing or speaking on a personal mobile electronic device,
a single-task being defined as either walking or performing the cognitive task;
a dual-task being defined as simultaneously walking and performing the cognitive task;
the device comprising:
an inertial sensor for collecting gait patterns of the person;
a touch input sensor for collecting touch-typing data or a microphone for collecting speech data;
an electronic data processor;
wherein the method comprises carrying out by the personal mobile electronic device the steps of:
   acquiring gait patterns from the inertial sensor and acquiring touch-typing data from the touch input sensor and/or speech data from the microphone,
   analysing the acquired gait patterns so as to obtain a single-task gait score and a dual-task gait score;
   analysing the acquired touch-typing data and/or speech data so as to obtain a single task cognition score and a dual-task cognition score;
   calculating a gait dual-task effect, gait DTE, score as a proportion between the dual-task gait score and the single-task gait score;
   calculating a cognition dual-task effect, cognition DTE, score as a proportion between the dual-task cognition score and the single-task cognition score;
   using the gait DTE and cognition DTE for assessing dementia-related disorders.

In an embodiment, the method further comprises tracking the gait DTE and cognition DTE over time for monitoring dementia-related disorders by determining changes in scores over a period of time.

In an embodiment, the method is for assessing cognitive decline and dementia; Alzheimer's disease and other related dementia; inclination to falls, instability, walking disability; gait and cognitive decline; or combinations thereof.

In an embodiment, the present disclosure describes a non-transitory computer-readable storage medium including program instructions for implementing a device for assessing dementia-related disorders of a person by monitoring cognitive task decline and walking gait changes as the person performs dual tasks in comparison with single-tasks, the program instructions including instructions executable by a data processor to carry out the method disclosed.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof.

The above described embodiments are combinable.

## Claims

1. A device for assessing dementia-related disorders of a person by monitoring cognitive task decline and walking gait changes as the person performs dual tasks in comparison with single-tasks, wherein the device is a personal mobile electronic device, and the cognitive task is typing or speaking on the device,
a single-task being defined as either walking or performing the cognitive task;
a dual-task being defined as simultaneously walking and performing the cognitive task;
the device comprising:
an inertial sensor for collecting gait patterns of the person;
a touch input sensor for collecting touch-typing data or a microphone for collecting speech data;
an electronic data processor configured for:
acquiring gait patterns from the inertial sensor and acquiring touch-typing data from the touch input sensor and/or speech data from the microphone,
analysing the acquired gait patterns so as to obtain a single-task gait score and a dual-task gait score;
analysing the acquired touch-typing data and/or speech data so as to obtain a single task cognition score and a dual-task cognition score;
calculating a gait dual-task effect, gait DTE, score as a proportion between the dual-task gait score and the single-task gait score;
calculating a cognition dual-task effect, cognition DTE, score as a proportion between the dual-task cognition score and the single-task cognition score;
using the gait DTE and cognition DTE for assessing dementia-related disorders.

2. The device according to the previous claim wherein the electronic data processor is further configured for tracking the gait DTE and cognition DTE over time for monitoring dementia-related disorders by determining changes in scores over a period of time.

3. The device according to any of the previous claims wherein the electronic data processor is further configured for detecting if the gait DTE goes below a predetermined gait DTE threshold or if the cognition DTE goes below a predetermined cognition DTE threshold to output a data record indicating a likelihood of a dementia-related disorder.

4. The device according to any of the previous claims wherein the electronic data processor is further configured for calculating a proportion between the gait DTE and the cognition DTE and for detecting if the calculated proportion goes beyond or below a predetermined DTE proportion threshold to output a data record indicating a likelihood of a dementia-related disorder.

5. The device according to any of the previous claims for assessing cognitive decline and dementia; Alzheimer's disease and other related dementia; inclination to falls, instability, walking disability; gait and cognitive decline; or combinations thereof.

6. A method for assessing dementia-related disorders of a person by monitoring cognitive task decline and walking gait changes as the person performs dual tasks in comparison with single-tasks, wherein the cognitive task is typing or speaking on
a personal mobile electronic device,
a single-task being defined as either walking or performing the cognitive task;
a dual-task being defined as simultaneously walking and performing the cognitive task;
the device comprising:
an inertial sensor for collecting gait patterns of the person;
a touch input sensor for collecting touch-typing data or a microphone for collecting speech data;
an electronic data processor;
wherein the method comprises carrying out by the personal mobile electronic device the steps of:
acquiring gait patterns from the inertial sensor and acquiring touch-typing data from the touch input sensor and/or speech data from the microphone,
analysing the acquired gait patterns so as to obtain a single-task gait score and a dual-task gait score;
analysing the acquired touch-typing data and/or speech data so as to obtain a single task cognition score and a dual-task cognition score;
calculating a gait dual-task effect, gait DTE, score as a proportion between the dual-task gait score and the single-task gait score;
calculating a cognition dual-task effect, cognition DTE, score as a proportion between the dual-task cognition score and the single-task cognition score;
using the gait DTE and cognition DTE for assessing dementia-related disorders.

7. The method according to the previous claim further comprising tracking the gait DTE and cognition DTE over time for monitoring dementia-related disorders by determining changes in scores over a period of time.

8. The method according to any of the claims 6-7 for assessing cognitive decline and dementia; Alzheimer's disease and other related dementia; inclination to falls, instability, walking disability; gait and cognitive decline; or combinations thereof.

9. Non-transitory computer-readable storage medium including program instructions for implementing a device for assessing dementia-related disorders of a person by monitoring cognitive task decline and walking gait changes as the person performs dual tasks in comparison with single-tasks, the program instructions including instructions executable by a data processor to carry out the method of any of the claims 6-8.
